# EUROPEAN PATENT SPECIFICATION

(11) **EP 0 612 500 B1**
(45) Date of publication and mention of the grant of the patent: **28.10.1998**
(21) Application number: 93911645.5
(22) Date of filing: 11.12.1992
(51) Int. Cl.: A61B 6/02, A61B 5/03, A61B 5/00

(54) **METHOD AND DEVICE FOR DIAGNOSIS OF LIVING ORGANISM**
VERFAHREN UND VORRICHTUNG ZUR DIAGNOSE VON LEBENDEN ORGANISMEN
PROCEDE ET DISPOSITIF POUR LE DIAGNOSTIC D'UN ORGANISME VIVANT

(30) Priority: 17.12.1991 SU 5020307
(43) Date of publication of application: 31.08.1994
(73) Proprietor: DYNAMICS IMAGING, Inc., Devon, PA 19333 (US)
(72) Inventor: Godik, Eduard Emmanuilovich, Suffern, NY 10901 (US)
(74) Representative: Ebbinghaus, Dieter, Dipl.-Ing.
(86) International application number: PCT/RU92/00237
(87) International publication number: WO 93/11704

(56) References cited:
- EP-A- 0 099 756
- EP-A- 0 108 617
- EP-A- 0 140 633
- EP-A- 0 290 273
- EP-A- 0 336 208
- WO-A-91/06244
- SU-A- 696 378
- SU-A- 1 482 654
- SU-A- 1 694 109
- SU-A- 1 711 817
- US-A- 4 446 871
- US-A- 4 832 035
- US-A- 4 846 183
- US-A- 4 960 126

## Description

The present invention belongs to the field of physics and medicine, or more concretely, to methods and apparatus, destined for obtaining information about physiological processes taking place in living organisms, it relates to a method of living organism state diagnostics and apparatus for its realization, which could be used for the investigation and diagnostics of functional dynamics of living organism physiological processes.

Functional diagnostics permits revealing the early precursors of pathology. This opens up the possibility of avoiding non-reversible disturbances in living organism and radical treatment methods. Due to this reason, methods of early functional diagnostics are acquiring increasing significance for organization of population screening and development of preventive medicine.

The state of living organism is reflected in continuous functional dynamics of its biological tissues. This dynamics is determined by functioning of the main distributed physiological systems, such as cell metabolism, supplying the tissues with energy, and microcirculation, supporting their metabolic resource. Functional status of these main life supporting tissue systems is determined by the distributed regulator mechanisms, both, the whole-organism, reflective and humoral ones, and the local, metabolic one. Cell metabolism is connected with micro-circulation via one else important distributed tissue system - perfusion. Its functional dynamics interconnects the first two systems.

The state of the whole-organism systems is reflected not only and not exclusively at the tissue functional dynamics at separate organism sites, but mainly by the degree and the character of the spatial connectivity of this dynamics. To explore this connectivity of tissue physiological processes, characterizing the whole-organism functional status, the investigation of continuous spatial distribution of functional dynamics of microcirculation is necessary. In the relaxation state, for example, spatial connectivity of the functional dynamics in the process of tissue activity is minimal, whereas it is greatly increased under the stress conditions; increased internal connectivity of the tissue functional dynamics is characteristic of cancer tumors as compared with surrounding normal tissues .

The spatial organization of the tissue functioning reflects the state of all the whole-organism systems and organs, which appeared during evolution just for supporting of the functional tissue status.

Each physiological parameter has its own physical image, physical "projection". Integral cell metabolism and microcirculation intensity in each organism site is reflected by its temperature value. In addition, one of the most informative physical parameters, reflecting the state of biological tissues of living organism, is their complex dielectric permeability. Its imaginary part, absorption coefficient of electromagnetic (EM) radiation by biological media, is characterized by a specific spectral dependence in the optical range for the main physiological agent of microcirculation - haemoglobin and its different functional forms: oxy-haemoglobin and carboxy-haemoglobin, as well as for cytochrome aa3, also participating in the cell metabolism. In a very wide range of EM-radiation frequencies, the real part of dielectric permeability depends on the degree of blood content in tissues and characterizes tissue inhomogeneity, produced, for example, by microcirculation dynamics.

In order to change the temperature and dielectric characteristics of biological tissues, different physical methods are applied for functional diagnostics investigations.

For non-invasive measurements of biological tissues temperature, their electromagnetic thermal radiation is registered. The latter is the most intensive in the middle infrared (IR) range, this make it possible to measure tissues temperature with the necessary accuracy of 0.1 degree. This is the essence of infrared dynamic thermovision method, which permits investigation of spatial distribution of living organism tissues functional dynamics.

In this method, temporary changes in spatial distribution of IR-thermal radiation intensity of living organism tissues are registered, reflecting their spatial-temporal microcirculation dynamics in the form of temporal sequence of thermoimages. This is performed both in the process of natural organism functioning and in the process of physiological reactions on different functional probes: reflective and humoral ones. Pain reactions, the influence of pharmaceutic treatments can be visualized via such procedure; the regions with different regulation disturbances can be revealed, differentially diagnosing which type of regulation is disturbed: reflective or humoral one; the state of internal organs can be estimated by specific reactions of the corresponding dermatomers (Zakharjina-Geda zones) etc. However, IR-thermovision method has a number of significant restrictions.

First, IR-thermal radiation carries direct information on physiological processes only in skin, since its characteristic absorption depth does not exceed 100 µm. Functional changes in blood content in skin capillary nets, disposed at the depth of 0.5 µm or larger, are reflected in skin temperature by means of thermoprojection, due to which a delay and inertia of several seconds emerges. That is why IR-thermal radiation does not reflect practically blood flow functional dynamics, connected, for example, with cardio- and respiratory pulsation, since thermoprojection time is several seconds.

Second, the temperature reflects blood flow and metabolism contributions simultaneously and integrally, which does not permit differential diagnostics of their disturbances.

Third, the middle IR-range technology, necessary for the realization of the above method, is unreasonably expensive.

For dielectric permeability measurements, the investigated part of the living organism is illuminated with EM-radiation and parameters of back scattered and/ or transmitted through biological tissues radiation are measured. EM-radiation of optical wavelength range - from 0.3 to 1.3 µm wavelengths - is effectively used for biological tissue investigation. At near IR wavelength range from 0.65 to 1.0 µm the tissues are transparent enough to be seen at depth of more than one centimeter. The transparence is limited by light scattering, the characteristic weakening length of such radiation makes up 3-5 µm for water-containing tissues. The absorption depths for the above mentioned physiological pigments reach up to several centimeters; in spite of the low absorption in the above region, it is selective enough to separate the contributions from the different pigments. For the wavelengths longer than 1.2 µm the transparence of water-containing tissues decreases sharply as a result of the strong water absorption.

Thus, photopletismography method is well known. The essence of this method is that the region under investigation, mostly the thinnest and thereby easier transilluminated body parts - ear lobes or hand and leg finger tips, are illuminated by EM-radiation of the above mentioned wavelength range; the intensity of transmitted and/or back scattered radiation is measured; the ratio of the latter intensities and that of the illuminating radiation (transmittance and back scattering coefficients, respectively) are calculated as a continuous functions of time; temporal dependencies of these coefficients, originating from cardiopulsation, are analyzed; functional state of microcirculation is judged from the extremum positions and the amplitudes ratio.

This method, however, is based on the analysis of the microcirculation functional mechanics at separate organism sites and gives no possibility to obtain and to estimate the organism surface, the spatial picture of its interconnection within the organism scope.

More weighty information on the functional microcirculation dynamics and cell metabolism in separate organism sites could be obtained using the method of spectral monitoring of biological tissues in the near IR-wavelength range (EP No. 0290278). To realize this method, the part of living organism under investigation is alternatively illuminated by radiation of several wavelengths in the range from 0.6 µm to 1.0 µm; the intensity of transmitted through the investigated field and/or back scattered radiation is measured for each wavelength as a continuous function of time; coefficients of transmittance and/or back scattered radiation are determined for each wavelength; the system of differential equations is solved with the use of these data and temporal dependencies of oxyhaemoglobin, carboxyhaemoglobin and cytochrome aa3 are calculated; the character of these dependencies is analyzed in the process of physiological reactions on different functional tests both local (cuff applying) and the whole-organism ones; thus obtained dependencies are compared with similar ones for a healthy person and the presence of pathology is judged by the deviations in the reaction amplitudes and the time delays. This method permits more detailed analysis of the functional state of the living organism to be performed: to reveal not only disturbances in the functional microcirculation mechanics, but also deviations in oxygenation status of the tissue investigated, in the ratio of arterial and venous blood content, in the perfusion state - by a delay in time dynamics and by the amplitude ratio of changes in oxyhaemoglobin and cytochrome aa3 concentrations. This extends significantly the possibilities of pathology revealing and its differential diagnostics.

However, this above described method as well as the previous one permits estimation of the character of functional dynamics of physiological processes only at separate organism points and does not give a possibility to visualize and to investigate a continuous spatial connectivity of these processes in the organism as whole.

Thus, currently used methods permit either investigation of spacial connectivity of the functional dynamics of physiological processes on the organism scale but with insufficient registration rate, insufficient probing depth and insufficient for differential diagnostics information on partial contributions of microcirculation and metabolic thermoproduction, or give the possibility of a detailed investigating pre-capillary and post-capillary blood flow and cell metabolism, but only in separate discrete points.

The method and apparatus are known which give the possibility of determining the optical properties of living organism tissues. To realize this method, the part of living organism under investigation, mammal gland, for example, is illuminated by electromagnetic radiation of optical wavelength range (near IR-range), transmitted through the organism radiation is taken and spatial distribution of its intensity is registered. This method opens up the possibility to reveal pathological inhomogeneities in transparency of living organism tissues but only of sufficiently large size and located not too deep from the surface, turned to the radiation detector. Tumors of mammal gland, for example, are possible to discover with method, as a rule, too late, when they reach already a large size.

EP-A-0 099 756 discloses a diaphanoscopy apparatus comprising means for sequentially illuminating an object with light of at least two different wavelengths, detector means for sensing transluminated light in relation to each sequential illumination, recording means for separately recording the output of the detector means related to the sequential illumination, and a display means which represents the absorption characteristics of the object at the different wavelengths. The apparatus according to EP-A-0 099 756 permits diagnostics of living organism state with the steps of illuminating the living oragnism or part of it with electromagnetic radiation of optical wavelength range and receiving transmitted through the living organism radiation of the optical wavelength range for such a diagnostic.

WO 91/06244 discloses a system and a method for measurement and presentation of fluid flow movements, particularly the flow of blood through a body organ. The system according to WO 91/06244 includes a laser beam generating source, means for directing the laser beam onto the body part to be examined and for moving the laser beam in a controlled fashion over the body part in accordance with a determined scanning pattern. This system further includes means for receiving part of the laser beam reflected from the body part. Also included in the system are means for detecting the broadened frequency of the reflected portion of the laser beam, this spectral broadening being caused by the Doppler effect, for a large number of points along the path scanned by the laser beam, this broadening of the frequency being used as a measurement of the magnitude of the superficial blood circulation in the examined body part at these points. In particular, WO 91/06244 discloses a method of permitting diagnostics of the living organism's state including the steps of illuminating the living organism or part of it with electromagnetic radiation of optical wavelength range and using such information for diagnostics.

It is an object of the present invention to provide a new method and apparatus for investigation of functional dynamics of physiological processes in living organism, which permits diagnostics of both tissue functional state and homeostasis stability of the living organism as whole via the character of continuous temporal-spatial distribution of physical parameters of the living organism.

This task is solved by the method according to which living organism or a part of it is illuminated by electromagnetic radiation of the optical wavelength range and transmitted though the organism and back scattered electromagnetic radiation of the optical wavelength range is received and registered in the form of image of spatial distribution of at least one parameter, characterizing transmittance and back scattering of electromagnetic radiation of optical wavelength range by the living organism, according to the invention, the above spatial distribution is registered sequentially and continuously in time. This gives the possibility of obtaining information on spatial distribution of functional dynamics of arterial and venous capillary blood content.

It is meaningful to register a spatial distribution of the transparence and back scattering coefficients of the electromagnetic radiation by the living organism.

The above electromagnetic radiation can be modulated by amplitude and transmitted and back scattered electromagnetic radiation receiving is performed then synchronously at the modulation frequency.

This permit to avoids the influence of a background light and the necessity of performing measurements in a specially darkened room.

The above mentioned electromagnetic radiation can be modulated by amplitude synchronously with one of the living organism physiological rhythms and to realize thereby stroboscopic registration of the back scattered or transmitted radiation.

This improves the visualization contrast of the pulse wave, for example.

To obtain information from different depth from the illuminated surface, the electromagnetic radiation is concentrated at least at one point of the living organism surface and radial distribution of the characteristics of back scattered and transmitted through the investigated field radiation are registered.

The surface of the illuminated living organism is scanned with the electromagnetic radiation, concentrated at least at one point.

This makes it possible to improve the spatial contrast.

Electromagnetic radiation, illuminating living organism, can be filtered by frequency in the range of physiological pigments absorption.

This gives possibility of separating the partial image dynamics, connected with microcirculation functioning and cell metabolism.

The frequency filtering can be performed at least for a one band from the wavelength range from 0.38 to 0.48 µm and/or from 0.52 to 0.62 µm, which permits separation of the image dynamics, produced by haemoglobin absorption from 0.6 um to 0.75 µm range, which permits separation of the image dynamics, connected with deoxygeneted haemoglobin absorption from the range of 1.0 µm to 1.1 µm, which permits separation of the image dynamics, connected with oxyhaemoglobin absorption.

Transmitted through the living organism and/or back scattered electromagnetic radiation can be filtered also by frequency in the range of absorption of physiological pigments, which permits, similar to the case of the illuminating radiation filtering, separation the partial image dynamics, connected with microcirculation functioning and cell metabolism.

In such a case, filtering can also be performed at least for a one band from the wavelength range of 0.38 µm to 0.48 µm and/or of 0.52 µm to 0.62 µm, which permits separation of the image dynamics, connected with haemoglobin absorption in the range of 0.6 µm to 0.75 µm, which permits separation of the image dynamics, connected with deoxygeneted haemoglobin in the range of 1.0 µm to 1.1 µm, which permits separation the image dynamics, connected with oxyhaemoglobin absorption.

The electromagnetic radiation can be modulated by frequency and transmitted through the living organism and back scattered radiation is received synchronously at the modulation frequency.

The electromagnetic radiation can be modulated by the frequency it least at one band from the wavelength range from 0.6 µm to 0.65 µm, which permits separation of the image dynamics, connected with oxyhaemoglobin absorption, and also from the wavelength range from 0.65 µm to 0.72 µm, which permits separation of the image dynamics, connected with deoxygeneted haemoglobin absorption. In the above described sequentially registered in time spatial distributions, the regions can be revealed, differing from each other at least by one of the parameters, characterizing their variation in time, and a set of the revealed regions can be registered in the form of functional map of physiological processes of the living organism.

This permits separation of the regions with a high correlation level of functional dynamics.

As a distinctive parameter, at least one parameter, characterizing transmittance and back scattering of the electromagnetic radiation at different points of the investigated region of the living organism, among them time cross-correlation coefficient, the velocity of the wave-like changes, the frequency of changes in time, time delay, the number and location of the extrema in temporal dynamics etc, is registered.

The registration can be performed synchronously, at lest with one of the natural physiological rhythms of the living organism. This permits to improve separation of microcirculation under the presence of radiation scattering from the surface layers of living organism.

As natural physiological rhythm of the living organism, breathing rhythm, rhythm of cardiopulsation and muscle tremor could be used.

Functionally connected living organism parts are to be mapped simultaneously.

This permits the revealing of the character of functional connectivity both between the regions, for example, between motor area of the brain cortex and skeletal muscles, internal organs and dermatoms reflectively connected with them, and also inside each of them. Paired organs of living organism are mapped simultaneously.
This gives the possibility of revealing abnormalities in nervous and/or humoral regulation, of finding out tumors which are characterized by a changed functional dynamics.

At least one external influence is applied in the process of illumination of a living organism or a part of it by the electromagnetic radiation.

The application of corresponding influences permits to reveal the separate contributions from the various physiological systems.

As external influences, changes in physical parameters of the environment, of living organism external breathing parameters, the composition of gas mixture breathed in by living organism, vibration, electric field, alternative magnetic field, physical exercises, ultrasound, alternative pressure and thermal radiation can be used.

This task is solved also by the fact that the apparatus, containing a source of optical radiation, a detector of radiation scattered by living organism, located at the living organism part which is opposite to the illuminated one, successively connected with a analog-to-digital converter, an input-output controller and a computer system, contains, according to the invention, an additional detector of back scattered by living organism radiation situated at the same side as the source of optical illumination does, a commutator, both inputs of which are connected with the outputs of the corresponding detectors of the scattered by living organism radiation, and the output to the input of the analogy-digital converter, three outputs of the controller being connected with the controlling inputs of the commutator and the scattered radiation detector, respectively, said apparatus recording transmitted and back scattered electromagnetic radiation of optical wave length range and recording maps of spatial distribution characterizing sequential and temporal continuous recordings of the transmitted and back scattered electromagnetic radiation.

The apparatus can contain a reference detector of the optical radiation, optically connected with the illumination source, and a divisor, included between the commutator output and an input of analogy-digital converter, the output of the referent detector being connected with the other input of the divisor.

The apparatus can contain successively connected sensor of at least one of the natural living organism physiological rhythms and temporal modulator, output of which is connected with an input of optical radiation and with corresponding input of input-output controller.

The sensor of natural living organism rhythms can be a detector of breathing rhythm, rhythm of cardiopulsation and muscles tremor of the living organism.

The apparatus can contain an optical system, located between optical illumination source and the living organism surface, focusing optical radiation at least at one point of the living organism surface.

The apparatus can contain a scanning system, optically connected with the optical one, whose input is connected with the corresponding output of the input-output controller.

The apparatus can contain at least one optical filter, located at the same optical axis as that of the optical illumination source and between the latter and the surface of living organism illuminated.

The apparatus can contain at least one optical filter, located at same optical axis as that of the detector of scattered by living organism radiation and between the above detector and the surface of living organism investigated.

The apparatus can contain a frequency modulator, the output of which is connected with the source of optical radiation, and the input - with the corresponding output of the input-output controller.

Below, the invention is explained by concrete modes of its realization and by the figures, in which:
fig. 1 is a general block diagram of a set-up for investigation of functional dynamics of living organism physiological processes, according to the invention;
fig.2 is a general diagram of a variant of a set-up for the investigation of functional dynamics of living organism physiological processes with normalization of the intensity of detected radiation, according to the invention;
fig.3 is a diagram of one of the possible construction variants of the reference detector;
fig.4 is a general block diagram of a variant of a set-up for the investigation of functional dynamics of living organism physiological processes with synchronization from the natural living organism physiological rhythms, according to the invention;
fig.5 is a diagram of one of the possible variants of the temporal modulator ;
fig.6 is a general block diagram of a set-up for investigation of functional dynamics of living organism physiological processes employing optical radiation focusing into separate points at the living organism surface, according to the invention;
fig. 7 is a diagram of one of the possible variants of the optical system ;

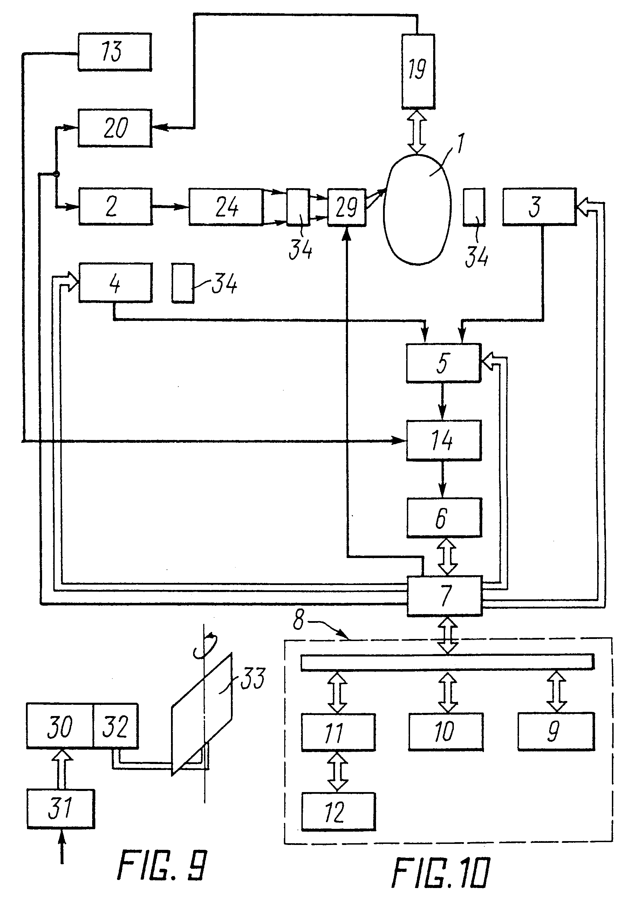

fig.8 is a general block diagram of a set-up for investigation of functional dynamics of living organism physiological processes with a scanning, according to the invention;
fig.9 is one of the possible variants of the scanning system ;
fig. 10 is a general block diagram of a set-up for investigation of functional dynamics of living organism physiological processes with filtering, according to the invention;
fig.11 is a general block diagram of a variant of a set-up for investigation of functional dynamics of living organism physiological processes with frequency modulation of optical radiation, according to the invention.

In order to realize the suggested method, a living organism or a part of it is illuminated with electromagnetic radiation of the optical wavelength range from 0.3 to 2.0 µm, the radiation transmitted through the living organism and back scattered by it is received and recorded in the form of images or maps of the temporal sequences of the spatial distributions at least one parameter, characterizing transmittance and back scattering of the radiation by living organism. For example, the intensity of transmitted and back scattered radiation is dependent on arterial and venous blood content. The degree of the optical radiation absorption by a healthy tissue and pathological one, tumor for example, is different, that is why the spatial intensity distributions carry information on the presence of tumors.

Living organism tissues are diffusively scattering media as optical radiation of optical range is concerned, and this considerably complicates the localization of the pathological inhomogeneities situated near the illuminated surface of living organism. Therefore, simultaneous registration of the intensity of back scattered radiation from the illuminated side and radiation transmitted through the region investigated gives additional information on the localization of pathological inhomogeneities, including tumors.

The above mentioned maps of the intensity spatial distribution are registered sequentially continuously in time, which gives information about temporal-spatial functional dynamics of arterial and venous capillary blood content. Under these conditions, the time of recording of each of such instant maps must be much less than the minimal time constant of the investigated physiological processes at the measured field or time intervals between their functional dynamics extrema. For securing continuous recording of such dynamical mapping in time, time interval between the maps must be much less than the minimal time constant of the investigated physiological process or time intervals between extrema of its functional dynamics, which should be larger, however, than the map recording time. Such a procedure is called dynamical mapping. For securing continuous registration of the each map all over the space investigated, the distance between the measurement points must be much less than the characteristic length of cross-correlation of the dynamic field investigated or the spatial scale of its variability. It should be noticed that discrete intervals for space and time, necessary for securing of continuous temporal-spatial imaging of living organism, in general are not independent of each other: if reflective processes - with the time constants of several seconds - have some certain value of the spatial cross-correlation length, then humoral ones - with the time constant of several minutes - have in general quite different one. The temporal sequence of images or maps obtained as a result of dynamical mapping we call by dynamical imaging or by dynamical mapping of living investigated.

As a parameter, characterizing transmittance and back scattering of the above mentioned radiation by living organism, the coefficients of transmittance and back scattering equal to the ratio of intensities of the transmitted and back scattered radiation and the illuminating one. In many cases, to reflect spatial-temporal dynamics of back scattering and transmittance of the radiation by the investigated region, it is convenient to use, instead of back scattering and transmittance coefficients, just the relative changes in the intensities of back scattered and transmitted through the investigated region electromagnetic radiation.

To exclude background light and the necessity of performing the measurements in darken rooms, the amplitude modulation of illuminating radiation and synchronous detection at the modulation frequency is applied, if necessary. The modulation frequency of the radiation intensity is chosen to be much larger of the natural physiological rhythms, including cardiopulsations. Under these conditions, a synchronous detection of the transmitted and back scattered radiation at the modulation frequency is used, The relative instability of the radiation amplitude modulation must be less than the relative changes in the radiation transmittance and back scattering coefficients.

The distributions of the radiation back scattering and transmittance coefficients are two-dimensional, plain ones. In majority of cases (except functional diagnostics of skin surface), functional dynamics distribution is necessary to be analyzed also as a function of depth of the investigated field.

In the diffusively scattering media, the biological tissues being among them for optical radiation of the near IR-range, deep layers give in the continuous spatial-temporal distribution of changes of the transmittance and back scattering coefficients the spatial frequencies of about the same scale as location depth of the dynamical sources. Therefore, by subsequent filtering ("cutting off") the more and more higher spatial frequencies, it is possible to separate at the dynamical maps the contribution of more and more deep layers.

The stroboscopic illumination, synchronous with different physiological rhythms of living organism: cardiopulsation, breathing, metabolic, thermoregulative ones, may be applied. The stroboscopic illumination for different organism parts can be delayed in time, for example, for reaching maximal possible contrast during pulse wave visualization.

For estimation of distribution of physiological processes functional dynamics over the depth, multilayer illumination of the region investigated is used, in particular .

The distribution over the depth of the functional changes in concentration of physiological pigments is reflected in radial around the illuminating beam distribution of the temporal dynamics of the radiation transmittance and back scattered coefficients. The relative contribution of more deep back scattering layers is increasing with increasing the distance from the beam. That is why the distribution over the depth is estimated by integral changes in the transmittance and back scattering coefficients as a function of alternative radius, r, Ktr(R) and Kbsc(R), respectively, in the radial region of around the beam. The procedure of normalization of transmitted and back scattered optical radiation intensity, taken as a function of R, by the intensity of the illuminating beam, which is performed during the calculation of Ktr(R) and Kbsc(R) automatically suppresses the inhomogeneities in the transmittance of the surface layers and the illumination instabilities. By increasing the number of discrete points ( the number of independent elements of the photodetector surface) within the radial region around the beam, the reliability of separation of the dynamics of physiological parameters over the depth is increased by a factor or a cluster analysis. Under these conditions the signal/noise ratio for each discrete element is correspondingly decreased. These oppositely acting modes are to be considered during optimization procedure of the spatial resolution of the photodetector system. The distance between the beams is limited by the requirement of continuous registration of the dynamic images over the space of the investigated region: it must be much less than the functional dynamics spatial correlation length, or otherwise, about ten or more points of the dynamical imaging must be located at the correlated functional dynamics regions. Practically, the distance between the beams is optimized taking into consideration the two oppositely acting modes - one for increasing the probing depth and the other for improving the spatial resolution.

It should be mentioned in addition, that as the distance from the illuminating beam is increasing, back scattered beams are coming from the larger depth, and the effective length L of their path through the light absorbing physiological pigments increases. By this reason, the modulation depth of the back scattered radiation also increases when the concentration of pigment (C), oxyhaemoglobin, for example, is functionally varied. Actually, the modulation depth is determined by the amplitudes of the absorption changes which is proportional to CL. This is also is taken into account when the distance between illuminating beams during dynamic mapping procedure is optimized.

As a simplified variant, one illuminating beam is applied in some cases. This variant is sufficient for investigation of the functional dynamics over the depth, which is important, for example, for estimation of the tumor location depth etc,. At the radius, corresponding to the location depth of a tumor, a considerable change in the temporal behavior of the back scattering coefficient must be observed due to changes in the character of microcirculation functional dynamics and metabolism of the pathological tissues.

Besides, scanning of the investigated region by one or several beams is used. The scanning can considerably improve the spatial contrast, but it is necessary to work with increased intensities, since under these conditions the signal/noise ratio is decreased due to a decrease in registration time of a spatial element of the region investigated. By increasing the number of simultaneously scanning beams, the signal/noise ratio is correspondingly improved and the registration time decreased. Scanning time for obtaining one instant frame is chosen to be much less than the time constant of the investigated process or less than the interval between extrema observed in its dynamics. For each point of the investigated region, from the temporal sequences of such frames, changes in transparence and back scattering at the equidistant points are chosen, thus obtaining radial spatial-temporal distribution of these coefficients. By the circle correlation of the temporal coefficients dynamics of different radial scale, the distribution of the functional dynamics over the depth of the investigated region is judged by.

To extract the partial maps dynamics, connected with microcirculation functioning and cell metabolism, spectral filters for investigation of selective absorption of oxyhaemoglobin, deoxygeneted haemoglobin and cytochrome aa3 are applied. In addition, spectral radiation selectivity in the above mentioned regions helps to exclude light scattering from the surface of the investigated region, which is only slightly wavelength dependent, under the conditions of measurements of the back scattered radiation, coming from the depth of the living organism. For separation of image dynamics, connected with microcirculation functional dynamics, haemoglobin spectral absorption bands in the wavelength range from 0.38 um to 0.48 um and/or from 0.52 um to 0.62 um are chosen.

To obtain spatial-temporal dynamics of arterial and venous blood content and cell metabolism in them, differential frequency modulation of the wavelength of the electromagnetic radiation, illuminating the surface of the living organism ( -modulation) is separately used, amplitude modulation of the dynamical maps being synchronously recorded, and frequency and phase signal selection at the modulation frequency and the doubled one being performed at the process of separation of corresponding changes at the dynamical maps. For the realization of maximal selectivity of the method, the wavelength, about which the modulation is performed, is chosen near the extremum and at the spectral intervals with maximal steepness. For example, to separate spatial-temporal dynamics of venous blood, the modulation wavelength interval from 0.6 µm to 0.75 µm is used, that for arterial blood - from 1.0 µm to 1.1 µm and for cytochrome aa3- from 0.85 µm to 0.9 µm. More concretely, for separating venous blood distribution dynamics of oxyhaemoglobin, deoxygeneted haemoglobin and cytochrome aa3, the modulation is performed about the following wavelengths: 0.7 µm - under these conditions the signal appears at the modulation frequency with the phase, corresponding to an increase in the back scattering coefficient together with the wavelength increase (let take this phase as zero one); 0.735 µm - in this case the signal is observed at the doubled frequency with the phase, corresponding to a decrease in the back scattering coefficient with deviation from the extremum; 0.745 µm - here the signal at the modulation frequency with the phase, opposite to that of the signal at 0.7 µm; 0.76 µm - here the signal with the doubled frequency modulation and the phase opposite to that of 0.735 µm signal. The modulation on these wavelengths which are characteristic of venous blood content can be used both separately and simultaneously. In the latter case multichannel correlative detection is applied, this increases greatly the recognition potential of venous blood distribution.

For separation of partial dynamics of arterial blood distribution, the modulation is performed near the following wavelengths: 0.815 µm , the signal at the modulation frequency with the phase p;; from 0.95 µm to 1.3 µm, the signal at the modulation frequency with the phase 0. The efficiency of separation of partial dynamics of the arterial blood distribution Increases under the conditions of simultaneous modulation at two above described spectral ranges and the employment of the correlative detection via two channels.

To isolate partial dynamics of cell metabolism, the modulation at the wavelength near the absorption maximum of oxidized cytochrome aa3 at 0.825 µm wavelength. When the modulation amplitude is high enough, + 0.025 µm , the signal is observed at the doubled modulation frequency with the phase, corresponding to the increase in the back scattering coefficient with deviation from the extremum.

It must be stressed that, together with the spectral selection, to separate the signals from the deep tissues in the presence of scattered from the surface radiation, the time independence of scattered light is used, since back scattered by deep tissues radiation is time modulated by functional dynamics of microcirculation and metabolism in them.

To reveal the functional connectivity of the field investigated, the measured temporal image sequences - dynamic image - are transformed with the help of mathematical treatment (factor, cluster or any other type of functional analysis) into a single map with separated regions of high correlation level of the functional dynamics or those with similar parameters of such dynamics (if cross-correlation of the whole field is high). Inside of the separated regions functional behavior of living organism tissues is mutually correlated. Such a map is called functional picture, functional image or functional map, and the corresponding procedure is functional mapping.

As a distinctive parameter in the functional mapping, the coefficient of cross-correlation of temporal dynamics of transmittance and/or back scattering coefficients. It must be stressed that, as has already been mentioned, the characteristic cross-correlation length, in general, is different for different physiological processes, for the mechanisms of its regulation, and varies with changing the living organism state. Therefore, the same living organism or a part of it is described by several different mutually supplemental functional maps.

A single or several functional maps are obtained as a result of analysis of tens and hundreds such maps, included into the temporal sequence of dynamical mapping procedure.

Functional maps of the living system are superimposed on its morphological image, obtained, for example, with the help of tomography, and thereby the spatial distribution of functioning is reflected.

A distinction of principle, of the functional mapping and traditional morphological one should be stressed. In the former case, one reveals and localizes the functonal "landscape" - the details of the functionally connected regions, while for the latter one the details of the spatial structure are of importance. As a rule, the characteristic dimension of the functional "landscape" (characteristic cross-correlation length) is much larger than the spatial size of discretization, therefore at the functional mapping such a high spatial resolution is not necessary.

Each physiological tissue system, microcirculation and metabolism, is described with its own peculiar set of the functional maps.

It should also be stressed that the functional maps, substituting tens and hundreds of maps of dynamic images, give the possibility of compact packing the information in the data banks, including a variety of early functional precursors of pathology.

Different regions with a high internal cross-correlation at the functional map are distinguished by the character of temporal dynamics of the back scattering and/or transmittance coefficients. There may be coefficient periodical changes , reflecting physiological rhythms of different time constant, amplitude, delay or relaxation changes in the process of physiological reactions of different time constant and amplitude, initiated by redistribution of blood content and metabolism rate in the process of vital activity.

In these cases, when a physiological process presents a spreading wave, as a distinctive parameter during the procedure of functional imaging, the velocity of wave-like changes in back scattering and/or transmittance of electromagnetic radiation is used. For revealing such wave - like processes at the dynamical maps, a method of time delayed cross-correlation, the delay being linearly dependent on the coordinate and variable in length dl/V (where dl is space discretization interval, and V -variable velocity, which is chosen so that to reach maximal cross-correlation. Such an approach is effective, for example, under the conditions of mapping of spreading depression waves in brain cortex. The velocity of these waves is very sensitive to changes in tissues, accompanying the development of tumors. In a similar way, the waves of blood content of bands can be mapped to reveal microcirculation disturbances.

As is distinctive parameters during separation of spatial regions, the frequency of temporal changes in transmittance and back scattering coefficients can be used, as well as time delay in the coefficients changing and the position of extrema of these coefficients.

Dynamical mapping of living organism is performed under different natural conditions: in sleeping and awaken states, before and after food taking etc. For each state its own set of the functional maps is characteristic which reflects microcirculation and metabolism dynamics. The aggregate of such functional maps for different states of living organism, put into the data bank, gives sufficiently complete description of the tissue functional status and permits revealing the earliest pathology precursors under the repeated investigation.

To separate the dynamical maps, reflecting microcirculation functioning, especially in back scattered radiation at the presence of background scattering from the living organism surface, a synchronous signal accumulation at the frequencies of natural physiological rhythms is applied.

As a source of reference signals, traditional electrophysiological sensors are used. This mode is used for separation of respiratory and cardiopulsation rhythms, muscle microtremor.

When natural respiratory rhythm is used as a synchronizing one, the functional maps reflect e.g. the distribution of respiratory pulsation amplitude and a delay (phase) of microcirculation blood content at oxyhaemoglobin absorption band in respect to the amplitude and phase of the respiratory rhythm. Living organism tissue functional state is reflected also in the dynamical maps of the relative amplitude and phase distribution of the respiratory rhythm at the spectral absorption band of cytochrome aa3, characterizing cell metabolism state. The dynamical maps of such a fast functional dynamics are reflecting, for the first time, the state of a reflective microcirculation regulation and must reveal the earliest signs of its disturbance.

The most effective mode for the dynamical microcirculation mapping is synchronous signal accumulation at the cardiopulsation frequency. As a reference signal, that of the photoplethismogram could conveniently be used, at the finger tip, for example. Variable at such registration delay (phase) depends, in principle, on the coordinate at the living organism surface. The velocity of the pulse wave can be estimated via this dependence.

The modulation amplitude of the tissue blood content by microtremor is very small, and rhythm is expressed only slightly, the signal is a noise-like. However, when the muscles are strained, this rhythm becomes more pronounced and its amplitude noticeably increases. The amplitude of such a modulation characterizes the state of the muscle tissue. This is an optical mode of tremor visualization. The modulation of blood content by microtremor characterizes an active reverse action of the muscle on its blood supply, venous flow out especially.

At functional imaging, contrary to the morphological one, functionally connected regions are necessary to investigate simultaneously. Only in such a way, it is possible to reveal the character of the functional connectivity not only between different regions, motor brain cortex and skeletal muscles for example, or internal organs and reflectively connected with them dermatomers - Zakharjina-Geda zones - , but also inside each of them. Of special importance at the investigation of paired organs is simultaneous functional mapping both of them. For example, hands and footsteps which are actively participate in thermoregulation and, therefore, are highly saturated with arterial-venous anastomosis. So, when the conductivity of nervous links is disturbed (e.g.at scattered sclerosis) a time delay of the functional dynamics is observed at hands and footsteps. Simultaneous mapping is very important under the investigation of mammal glands. An asymmetry in the functional maps with different time scales of such organs may indicate on the disturbances of nervous and/or humoral regulation and on tumor appearance.

The most valuable information could be obtained via dynamical mapping of physiological reactions of living organism when some external influences are applied. With the help of the corresponding choice of the influences the contribution of different physiological systems could be accented. For example, variation of the temperature of the environment initiates thermoregulation reactions of microcirculation in skin, short time physical exercising tests the muscles microcirculation state, while more prolonged ones - cell metabolism functional state in muscles; reflective influences (pain, emotional ones etc.) reveal the state of nervous regulation; changes in the composition of air-the oxygenation tissue status. Different functional states of living organism, are differentiated by the spatial-temporal organization of the optical dynamic maps, reflecting transition processes of the tissue physiological reactions. Depending on the state of living organism, different types of such processes are observed: relaxation, oscillation, wave like ones. Taking into consideration that each type of the transition processes is described by a set of quantitative parameters (amplitudes, relaxation times, periods, phases, delays etc.) and also that optical dynamical maps are registered simultaneously at several spectral ranges (in order to characterize the behavior of different physiological pigments), optical functional imaging has a great potential for recognition of the earliest pathological changes in the functional state of living organism.

It is important that chosen external influences were close to the natural ones, living organism having been adopted to in the course of evolution: those connected with breathing, taking food, physical exercises, thermoregulation, nervous reactions. Two types of the functional maps are investigated. The first one characterizes physiological reactions of living organism tissues on relatively small (differential ones) external influences, e.g. light physical exercises, changes in the composition of breathed in air, external temperature within the comfortable range etc..Such functional maps characterize the functional organization and the state of the tissue physiological systems within the limits of its natural comfortable physiological reactions. The structure of these functional maps is practically independent of the amplitude of external influence, such dependence is observed only for the amplitude changes of the radiation transmittance and back scattering coefficients at the dynamical maps. The second type of functional maps in obtained for sufficiently large external influences intensities, when the structure of the functional maps changes. The intensities of external influences, at which such changes appear , characterize the dynamical ranges of the physiological reactions of living organism tissues, and therefore, such maps reflect the whole-organism stability of the living system.

As natural external influences, changing of external physical conditions is used: temperature, humidity, external electrical field, vibrations and others.

Changes in temperature, thermal flows, humidity, controlling the evaporation rate from the living organism surface, initiate physiological reactions of the thermoregulation system which are provided, primarily, by skin microcirculation functioning. Under these conditions, functional state of skin microcirculation network is reflected at the corresponding dynamical and functional maps.

Functional state of living organism tissues is determined by their oxygenation, which depends on the parameters of external breathing: its frequency, depth, the ratio between the breathe in and breathe out length. By this reason these parameters are used as external influences during the functional mapping of living organism. Both fast temporal dynamics with the time constant of less than one minute, and more slow humoral one are observed under these conditions. The fast one is observed, for example, at breathe holding. The time of recovery the tissue parameters after breaking off the external influence characterizes total stability of the organism homeostasis: the shorter this time, the higher organism stability.

One of the most strong natural external influences is a change in the composition of breathing in air. For this purpose both enriched with oxygen mixtures, and oxygen deficient or enriched with CO2 and/or nitrogen mixtures are used. Under these conditions, a characteristic redistribution of the values of optical radiation transparence and back scattering coefficients in the spectral bands of oxy-, deoxyhaemoglobin and cytochrome aa3 is observed. The earliest stages of obstruction (lung deficiency) are seen both as peculiarities in the spatial-temporal distribution of the above mentioned coefficients, and as an increase in the recovery time after cessation of the influence action.

Vibration, applied as an external influence, reveals at the dynamical maps functional reactivity of microcirculation connected with mechanoreception. Dynamical and functional maps of living organism microcirculation reactions depend on the vibration influence frequency, in accordance with the frequency dependence of mechanoreceptors. At such maps, the regions with abnormal temporal dynamics of the optical radiation transmittance and back scattering coefficients at the spectral absorption bands of oxy- and deoxyhaemoglobins are seen as the more contrast ones, as compared with the surrounding tissues and with statistically averaged data. Changes in microcirculation initiate also corresponding reactions of the cell metabolism which are reflected at the dynamical maps of optical radiation transmittance and back scattering coefficients at the absorption band of cytochrome aa3.

The comparison of these two types of the dynamic functional maps permits the estimation of the perfusion state. Under the conditions of frequency variable vibrational influence, dynamical maps at the cytochrome aa3 absorption band reflect also the characteristic frequency dependence (frequency range from 8 to 30 MHz) of the cell metabolism rate.

Electric field, taken as an external influence, affect on the non-specific skin reception and reflectively provoke microcirculation skin network reaction. Skin microcirculation has been found out to "mark" external electric fields, constant and alternative ones, beginning with relatively small ones of about 1000 V/cm. This sensitivity depends significantly on the voltage distribution across living organism surface, as well as on the average frequency, the character of its temporal variability and the length of acting upon.

The functional maps, obtained under the physical exercises conditions, reflect, primarily, the state of the working muscle tissues. At first, they reflect the character of metabolic resource utilization, then (after 10-20 s) changes in microcirculation are added as well as associated processes of metabolic support of energy losses in muscles. In such a way, via time delay in the functional maps, metabolic resource is estimated, as well as the possibilities of microcirculation supply of the muscle functioning, perfusion state and cell metabolism. It is worth noting that under the physical exercises of one from two pair muscles, a physiological reaction is observed also for the other one, and, in addition, practically the whole muscle system responds. The functional map of the latter system reflects the character of a reflective connection between the muscles and also humoral reactions.

Ultrasound, as an external influence, also permits initiation of internal organs and tissues, but with much more high spatial directionality. By scanning an organ with ultrasound beam, pathologies can be revealed and localized via the anomalies appearing in the functional maps of the referent dermatomer.

When alternative magnetic field with the frequency of tens to hundreds kilohertz, for which biological tissues are transparent, is applied as an external influence (in the form of exciting currents), it initiates reaction of the internal organs: liver, kidney and other organs. Under these conditions, the reflective and humoral projections (Zakharjina-Geda zones) of the affected organs are revealed at the functional maps of the presurface tissues reflecting both the state of these organs and their connection with the referent dermatomers.

Living organism tissue functional state is clearly revealed at the functional maps under the application of external pressure. Under these conditions, changes in both blood content and reological characteristics of tissues are observed as a result of changes in correlation of the radiation scattering inhomogeneities. The changes in blood content and tissue reology can be separated, taking into consideration that blood content changes are spectrally dependent, while reological ones practically do not. The most informative are functional maps, reflecting transition processes of tissue physiological reactions on a jump - like pressure application and on taking it off. The jump - like pressure application and taking it off means that the corresponding time must be much less than time interval of transition physiological reaction in answer to pressure application and taking off. A choice of time interval between pressure application and taking it off is also important, it must be larger than that of establishing of the tissues stationary physiological state after pressure application. Time of establishing of physiological tissue status after pressure application and taking it off is of a separate diagnostic significance. This time is taken to be equal to that of reaching by initial physiological pigment concentration of its stationary level with the accuracy of 10%. Investigation of continuous spatial distribution of the continuous temporal changes in the radiation transmittance and back scattering coefficients, when using pressure as an external influence, permits revealing of regions with disturbed microcirculation functional dynamics: with amplitude deviation from normal state, time constant of physiological reactions, the recovery times of the stationary physiological tissue status after physiological reactions. Pressure, as external influence, effectively reveals mammal gland inhomogeneities, including the early stages of cancer.

Thermal flows of about 1 mW per square centimeter affect noticeably on non-sensible perspiration rate, as one of the main living organism thermoregulation mechanisms. Skin microcirculation is also changed under these conditions. By this reason, it is quite natural to use such thermal flows as an external influence at the organism functional mapping. The intensity of the affecting thermal flow is time varied and redistributed across the living organism surface. The functional maps of living organism are studied as a function of the stimulus spatial-temporal organization, e.g. under the influence of this stimulus on one of the hands or footsteps. The most informative are the functional maps, reflecting the reactions on fast (as compared with the reversed reaction time) switching on/switching off or thermal flow redistribution. In particular, instead of a heating flow, a cooling one can be used: it is enough for this purpose to place near the living organism a surface having the temperature below that of the environment.

### BEST MODE FOR CARRYING OUT THE INVENTION

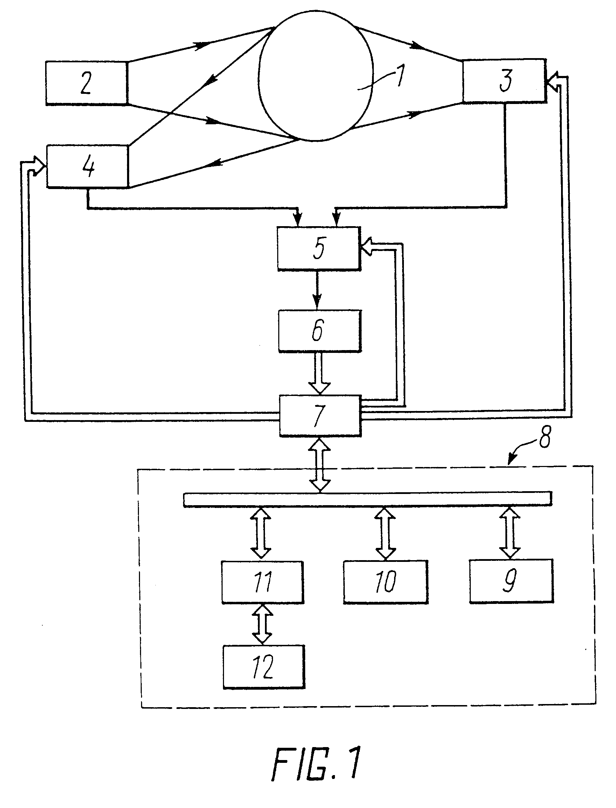

In accordance with figure 1, living organism surface 1 is illuminated with a source 2 of optical radiation. The spatial distribution of the intensity of transmitted and back scattered radiation is converted respectively by detector 3 and 4 of scattered by the living organism radiation into electrical signals, coming to the corresponding inputs of a commutator 5. As detectors 3,4 of the scattered by the living organism radiation, television camera on coupled charge device can be used, since it is characterized by a high resolution and sensitivity at the near IR-range. Commutator 5 omits sequentially and alternatively, for example, through one frame signals from the outputs of detectors 3 and 4 to the analog-to-digital converter 6. Digital signal via input/output controller 7 comes the computer system 8, where is stored in memory unit 9 as maps sequence, which can be displayed, with the help of processor 10 and display controller 11, at display monitor 12. To extend the dynamical range, processor 10 may put the corresponding color in accordance with a definite range of the signal level.

The temporal map sequences thus obtained are mathematically treated, analyzed with factor, cluster and/or other type of functional analysis. Such treatment converts temporal map sequence into a single map, separating the regions with a high level of the functional dynamics correlation or with proximity of such dynamics parameters. As a distinctive parameter, the cross-correlation coefficient of temporal dynamics of the transmittance and/or back scattering coefficients can be used for separation of the interconnected regions. In this case, mathematical treatment at the processor system comes to the calculation of intercorrelative functions between temporal dependencies of the transmittance and back scattering coefficients, relating to the map separate spatial points. A group of points with maxima of the intercorrelative functions differing by not more than some definite value are united into a single region and are marked by the same color when displayed at the monitor, The other group of points, also satisfying to the aforementioned requirement, are marked by the other color and etc.. Due to such procedure the temporal sequence of spatial maps is converted into a single functional map (functional image).

This mathematical treatment provides a good separation of slowly changing signals at the presence of background noises which were not seen at the initial maps.

Input-output controller 7 may contain one or several known controllers, securing information input and output from the computer system 8. Into the input/output controller 7 analog-to-digital converters are included, their number being equal to the number of the analog outputs destined for transformation of digital codes into the analog signals.

Input/output controller 7 transfers synchronized scanning signals and the signal of charge transfer control from the section accumulating radiation scattered by living organism into the storing one of detectors 3 and 4 (for the case of usage of television camera for coupled charge devices). The latter signal permits changing accumulation time at the target by a software, thereby increasing the signal/noise ratio. Input/output controller 7 controls also switching over commutator 5, including, e.g. a counter trigger. A commutator controlled by a digital code may also be used, but in this case, a meter is set up at the commutator input (this meter is included into commutator 5). In a general case, several control signals are brought to detector 3,4 of the scattered by living organism radiation and to commutator 5, due to this reason the corresponding connections are shown as tires.

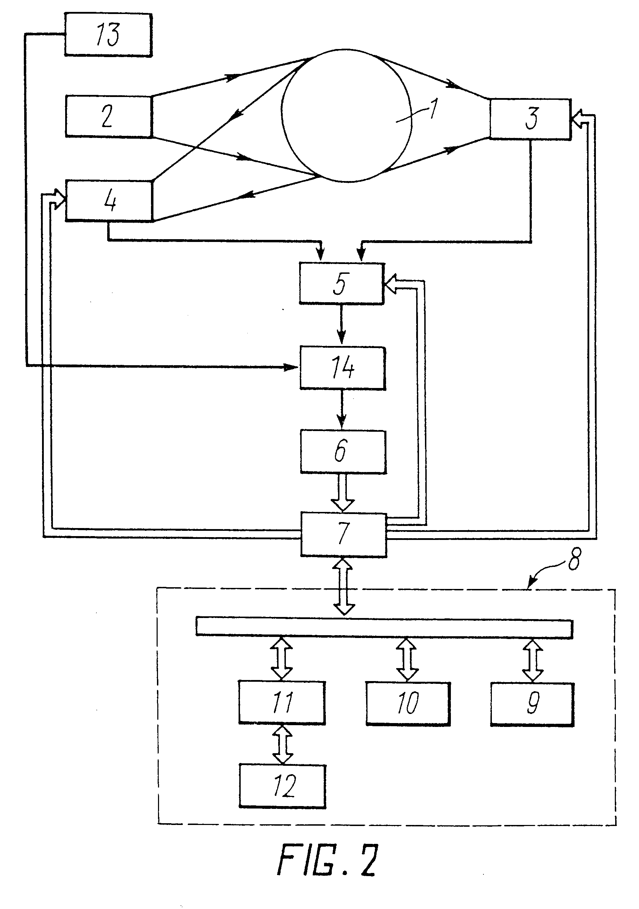

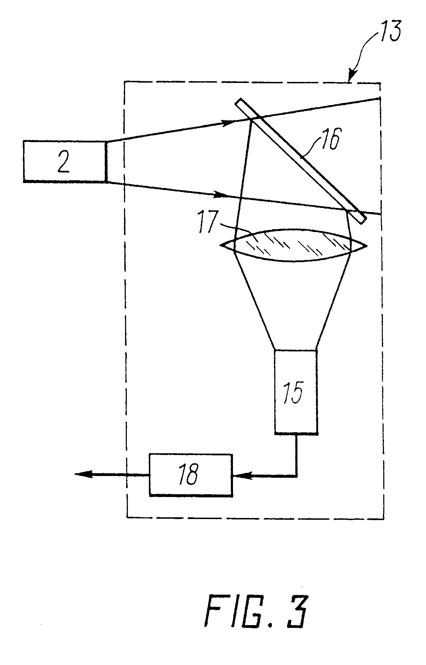

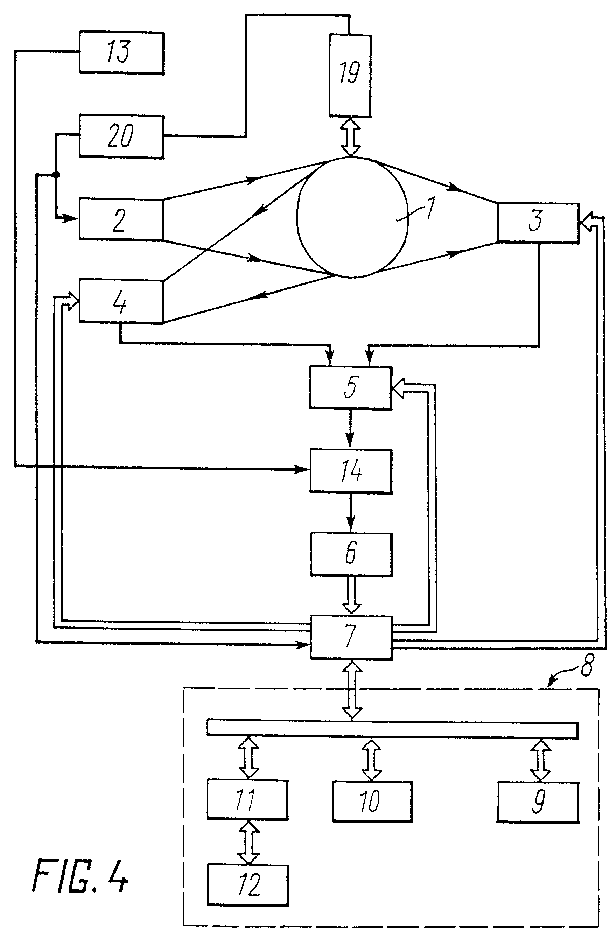

To avoid temporal instability of source 2 of optical radiation and inhomogeneities in the distribution of illuminating intensity, the apparatus contains additionally (figure 2) reference detector 13, optically onnected with source 2 of optical radiation, output of which is connected with divisor 14, inserted between commutator 5 output and analog-to-digital converter 6.

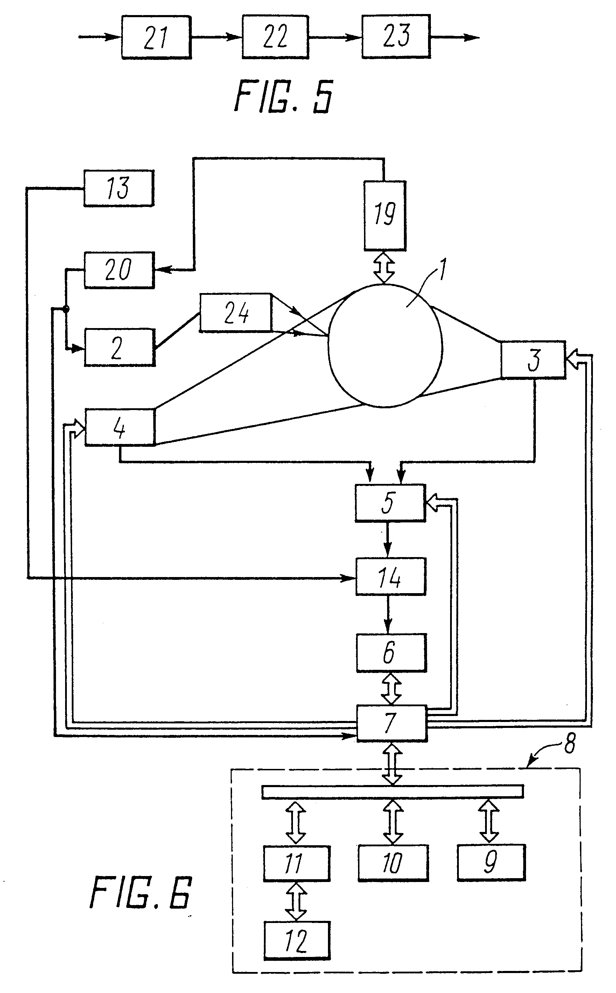

One of the possible variants of the reference detector realization is shown in figure 3. The reference detector may represent photodetector 15, optically connected via semitransparent plate 16 and lens 17 with source 2 of the optical radiation. Light is reflected from semitransparent plate 16 and is focused by lens 17 to photodetector 15, where it is converted to the electric signal and amplified by amplifier 18.

The transmittance band of amplifier 18 is chosen to be proportional to the inverse value of the signal accumulation time at detectors of scattered by living organism radiation. The output signal of amplifier 18 is proportional to the radiation intensity, coming to the living organism, and is received by divisor 14, normalizing the output signal of detectors 3,4 of radiation scattered by the living organism.

An amplifier with controlled amplifying coefficient, widely used at set-ups of automatical amplifier control, may be used as divisor 14.

It is worth noting that, in most cases, to diagnose the living organism functional state, not absolute values of coefficients K of transmittance and back scattering of the optical radiation are of importance, but only their relative changes in time, $\text{DK/K = DI/I}$, where I is the intensity of transmitted or back scattered radiation. This relative value is practically independent of the illumination inhomogeneity and temporal instability (under the condition that interframe interval is less than the characteristic time of the illumination instability. In such a case, reference detector 13 and divisor 14 may be not necessary.

As mentioned above, to separate the dynamical maps, reflecting microcirculation functioning, under the conditions of insufficient illumination stability and without proper control of living organism surface 1 movements during measurements, a stroboscopic pulse illumination , synchronized with one of the natural living organism physiological rhythms (breathing, cardiopulsation etc.), modulating microcirculation, is used. For this purpose, the apparatus (fig. 4) contains sensor 19 of natural physiological rhythm, which could be realized, depending on the concrete task, via known and widely used in medical practice meters of breathing rhythm as well as of cardiopulsations and muscle tremor.

The output signal from sensor 19 of physiological rhythm is received by temporal modulator 20. This modulator may be realized according to scheme of figure 5. It contains sequentially connected Shmitt trigger 21, generator 22 of delay and generator 23 of the modulating pulse. Let consider the temporal generator functioning when a pulse wave is brought at its input. A rectangular pulse with an abrupt front part is formed by Shmitt,s trigger from the initial pulse signal. Generator 22 of delay forms a pulse whose front is connected with that of the signal from Shmitt,s trigger output and whose duration is determined by a necessary delay. This pulse signal comes to generator 23 of modulating pulse input, where a pulse is formed, the forefront being connected with the back front of the input signal and the duration being determined by the task under investigation, as a rule, not longer than several percents of the pulse signal period. The pulse signal from temporal modulator 20 comes to 10 source 2 of optical radiation and to input-output controller 7. Source 2 of optical radiation operates only during pulse signal duration. Bringing the signal from modulator 20 output to input-output controller 7 is necessary for synchronization of the process of digitizing the signal from detectors 3,4 of the scattered by living organism radiation.

For the case when television camera based on the coupled charge device is used as detectors 3,4 of radiation scattered by living organism, the synchronization is carried out in such a way that charge transfer from the accumulative section to the storing one proceeds just after the termination of the pulse signal of the temporal modulator 20.

The variant of the apparatus shown in figures 4 and 5 may be used also for separation of the dynamical maps, reflecting microcirculation functioning, of the back scattered radiation (recorded with detector 4), especially, at the presence of a background scattering from the surface of living organism, not containing blood vessels layers, and under the continuous illumination. In this case, the synchronizing signal from sensor 19 of the natural rhythm through temporal modulator 20 is brought only to input-output controller 7 for digitizing of signals of detectors 3,4 and for the synchronous frame accumulation into memory unit 9 of computer system 8.

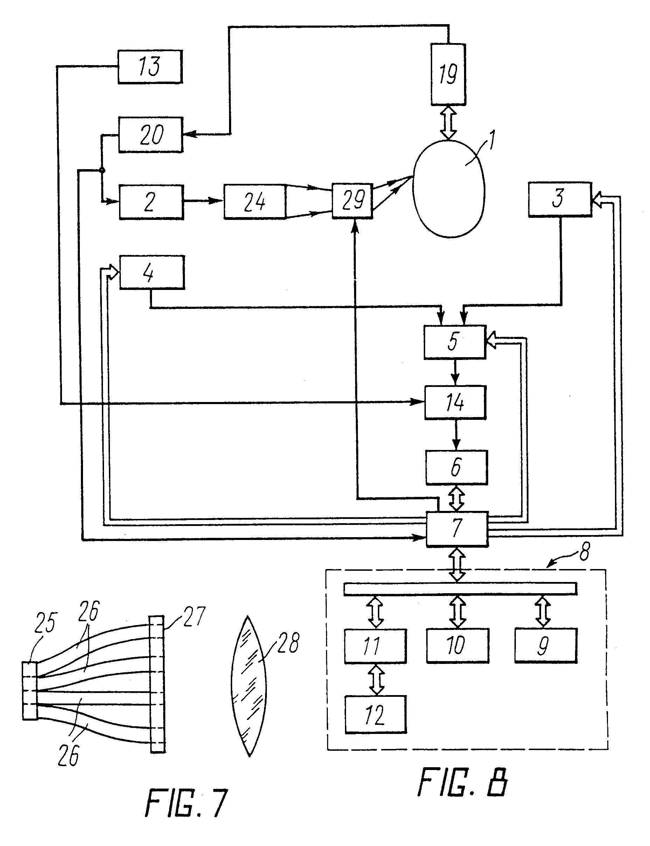

For separation of the dynamical maps, characterizing living organism tissues, located at different depths, as aforementioned, the illuminating radiation is focused at least at one surface point and a radial distribution of the scattered around the beam light is registered. To provide such focusing of optical radiation, optical system 24 (figure 6) is included between the source of optical radiation and living organism. In the simplest case of radiation focusing to one point, the optical system may be presented by a zoom lens.

To obtain several focused beams, an optical system, shown in figure 7, may be used. It consists of disk 25, placed in the plane of the aperture of the optical radiation source, fiber optic cables 26 being fixed in it. The number of fiber optic cables 26 is determined by the number of points at living organism surface, the radiation is focused to. The other ends of fiber optic cables 26 are fixed at the surface of the other disk 27 at some distance from each other, which is determined by mutual disposition of the necessary points at the surface of living organism. The images of the fiber optic cable torsions with the help of zoom lens 28 are projected at the living organism surface.

For scanning of the illuminated points at the living organism surface, a scanning system 29 (figure 9) is included between the optical system and this surface. The scanning system consists of driver 30, scanning control block 31, reductor 32 and mirror 33. The latter is located at the optical axis of the optical system 24 and is mechanically connected via reductor 32 with driver 30, which is connected with the output of scanning control block 31. The most purposeful is usage of discrete beam (set of beams) capable of shifting across the surface of living organism.

In this case, a stepwise driver may be used as driver 30 ; and to turn it by one step, the corresponding pulse voltages are brought at the stepwise driver from scanning control block 31. To synchronize the scanning process with the functioning of the apparatus as whole, with detectors 3,4 of scattered by living organism radiation, in particular, the control input of the scanning controlling block 31, is connected with the corresponding output of input-output controller 7.

To perform spectral filtration of the illuminating radiation and/or that of the transmitted through living organism and back scattered from its surface , which is necessary for separation of partial image dynamics connected with microcirculation and cell metabolism, near aperture of optical radiation source 2 and/or near detectors 3,4 of radiation scattered by living organism at least one optical filter is placed (figure 10).

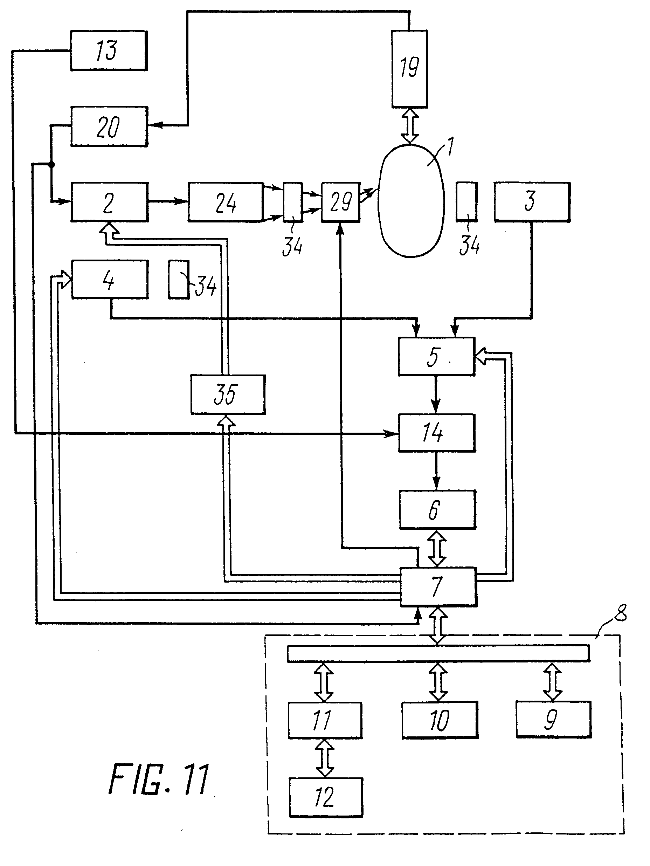

As mentioned above, the frequency modulation of radiation illuminating living organism, gives the possibility of obtaining partial dynamic maps, characterizing separately the functional dynamics of oxy-, deoxyhaemoglobin and cytochrome aa3 concentration changes. For this purpose, the apparatus contains frequency modulator 35, connected with source 2 of optical radiation (figure 11). In one of the possible apparatus variants, the frequency modulator can be realized as a set of controlled current sources. Each of the controlled current sources provides energy supply for one or several groups of light emitting diodes, located at optical radiation source 2. Each group of the diodes radiates light at its own wavelength.

Change in optical radiation wavelength is provided by sequent switching on corresponding controlled current sources. The control signals come to the frequency modulator from the corresponding output of input-output controller 7.

### Industrial application

Therefore, the above described apparatus for diagnostics of living organism functional state as well as the prototype contains a source of optical radiation, a detector of transmitted radiation which is located at the opposite side from the illuminated part of living organism, sequentially connected to an analog-to-digital converter, an input-output controller and a computer system. It contains an additional detector of back scattered radiation, located at the same part of living organism as the radiation source and a commutator, which give the possibility of sequential registration, connecting to the analog-to-digital converter and to the subsequent recording tract, signals from both detectors of transmitted and back scattered radiation. Such two-channel registration of scattered by living organism radiation gives the possibility sufficiently improve spatial resolution of revealing pathological tissue inhomogeneities, tumors for the first turn, which are located near the illuminated surface, and also those, which are located deep enough from both surfaces of living organism. However, the main advantage of the apparatus is that it works in a quite different regime: instead of only spatial distribution (one frame) as in the prototype, in this apparatus, with the help of analog-to-digital converter and input-output controller in the memory unit of the computer system, the continuous sequence of frames is accumulated. To secure continuous registration in time, with the help of a computer via an input-output controller the intervals between the frames are set to be much less than the time constant of physiological process investigated. The commutator gives the possibility of the frame registering at the transmitted and back scattered radiation alternatively, thereby practically simultaneous images recording of living organism in the scattered light from the both sides is achieved. Such dynamic living organism images recording permits, as aforementioned, revealing the early stages of pathologies, including cancer tumors, since before a tumor achieves the size enough to be distinguished at morphological mapping (one frame) it may change sufficiently the character of functional dynamics of blood content microcirculation at much more extent region of living organism. This may be revealed at dynamic maps of living organism both under its natural variability and, especially, under the reactions on various external influences.

The method and the apparatus are absolutely harmless and give the possibility of obtaining functional image of living organism, reflecting varied spatial-temporal organization of physiological reactions of microcirculation and cell metabolism, which permits perceiving various disturbances in physiological regulation at the earliest stage of its revealing.

## Claims

1. The method of diagnostics of living organism state, comprising the steps of:
illuminating living organism or a part of it with electromagnetic radiation of optical wavelength range,
receiving transmitted through living organism and back scattered radiation of optical wavelength range,
recording it in the form of maps of spatial distribution of at least one parameter, characterizing transmittance and back scattering by living organism of electromagnetic radiation of optical wavelength range, the aforementioned spatial distribution being recorded sequentially and temporally continously.

2. A method as described in claim 1, characterized by recording the spatial distribution of the coefficients of transmittance and back scattering of the aforementioned radiation by living organism.

3. A method as described in claims 1,2, characterized by amplitude modulation of the aforementioned radiation.

4. A method as described in claims 1-3, characterized in that the aforementioned electromagnetic radiation is stroboscopically modulated by amplitude synchronously with one of the living organism physiological rhythms.

5. A method as described in claims 1-4, characterized by a concentration of electromagnetic radiation at least at one point of the living organism surface and recording a radial distribution relative it of at least one parameter, characterizing back scattering of this electromagnetic radiation by living organism tissues and, thereby, the state of the organism tissues at different depths is judged by.

6. A method as described in claim 5, characterized by scanning the illuminated surface of living organism with the aforementioned electromagnetic radiation, concentrated at least to one point.

7. A method as described in any of the above claims, characterized by spectral filtering of illuminating living organism electromagnetic radiation, transmitted through and/or back scattered by it, at the physiological pigments absorption bands.

8. A method as described in claim 7, characterized by a frequency filtering of the radiation at least at one band from the wavelength interval from 0.38 µm to 0.48 µm and/or from 0.52 µm to 0.62 µm.

9. A method as described in claim 7, characterized by the radiation frequency filtering at least at one band from the wavelength interval from 0.6 µm to o.75 µm.

10. A method as described in claim 7, characterized by the radiation frequency filtering at least at one band from the wavelength interval from 1 µm to 1.1 µm.

11. A method as claimed in any of the above claims, characterized in that the frequency modulated transmitted through and back scattered by the living organism electromagnetic radiation is received synchronously at the modulation frequency.

12. A method as described in claim 11, characterized by modulation of a beam at least at one band from the wavelength interval from 0.6 µm to 0.65 µm and/or 0.65 to 0.72 µm.

13. A method as described in any of the above claims, characterized by revealing in sequentially recorded aforementioned spatial distributions spatial regions, differing from each other by at least one parameter, characterizing their temporal change, and by recording of the above regions in the form of a functional map of physiological processes taking place in living organism.

14. A method as described in claim 13, characterized by using, as a distinctive parameter, the temporal cross-correlation coefficient of at least one parameter, characterizing transmittance and back scattering of the aforementioned electromagnetic radiation at different points of the region of living organism investigated.

15. A method as described in claim 13, characterized by using as a distinctive parameter, under investigation of wave-like physiological processes, the velocity of temporal wave-like changes of at least one parameter, characterizing transmittance and back scattering by living organism electromagnetic radiation is used.

16. A method as described in claim 13, characterized by the use, as a distinctive parameter, a frequency of temporal changes of at least one parameter, characterizing transmittance and back scattering of electromagnetic radiation by living organism.

17. A method as described in claim 13, characterized by the use, as a distinctive parameter, a temporal delay of changes of at least one parameter characterizing transmittance and back scattering of the aforementioned radiation by living organism.

18. A method as described in claim 13, characterized by use, as a distinctive parameter, the number and position of extrema at the temporal dynamics of at least one parameter, characterizing transmittance and back scattering of the aforementioned radiation by living organism.

19. A method as described in any of the above claims, characterized by a synchronous recording with at least one of the natural living organism physiological rhythms.

20. A method as described in claim 19, characterized by using of living organism breathing rhythm, as a natural physiological rhythm of living organism.

21. A method as described in claim 19, characterized by using living organism cardiopulsation rhythm, as a natural physiological rhythm of living organism.

22. A method as described in claim 19, characterized by using living organism muscle tremor, as a natural physiological rhythm of living organism.

23. A method as described in any of the above claims, characterized by a simultaneous investigation of the functionally connected regions of living organism.

24. A method as described in any of the above claims, characterized by simultaneous investigation of living organism paired organs.

25. A method as described in any of the above claims, characterized by application of at least one external influence on living organism or a part of it.

26. A method as described in claim 25, characterized by the use, as an external influence, of changes in physical parameters of the environment, surrounding living organism.

27. A method as described in claim 25, characterized by use, as an external influence, of changes in the parameters of external living organism breathing, and via obtained optical functional maps, characterizing physiological reactions of living organism, the state of both respiratory system and the whole-organism homeostasis being judged by.

28. A method as described in claim 25, characterized by use of vibration, as an external parameter.

29. A method as described in claim 25, characterized by use of an electric field, as an external influence.

30. A method as described in claim 25, characterized by use, as an external influence, of alternating magnetic field, affecting different regions of an organ investigated, and via the optical functional maps of physiological reactions on such an influence at the reflective skin projections of this organ, pathology presence and localization being judged by.

31. A method as described in claim 26, characterized by use of physical exercise, as an external influence.

32. A method as described in claim 26, characterized by use, as an external influence, of ultrasound focused to at least one point of diagnosed internal organ, and the presence and the localization of pathologies being judged by the optical functional maps of physiological reactions on this influence at the corresponding to this organ dermatome.

33. A method as described in claim 25, characterized by use of external pressure as external influence, and the state of diagnosed organs being judged by the optical functional maps of physiological reactions on its application and/or removal.

34. A method as described in claim 25, characterized by use of thermal radiation and/or thermal flows, as an external influence.

35. An apparatus for diagnostics of living organism state containing: a source (2) of optical radiation; a detector (3) of scattered by living organism (1) radiation located at the opposite side from the illuminated part of living organism (1), sequentially connected analog-to-digital converter (6), an input-output controller (7), a computer system (8), an additional detector (4) of scattered by living organism radiation located at the same side as the optical radiation source (2), commutator (5), two inputs of which are connected with outputs of corresponding detectors of scattered by living organism radiation, while the output - to analog-to-digital converter (6) input, three outputs of input-output controller being connected with control inputs of, correspondingly, the commutator and the detector of scattered by living organism radiation, said apparatus recording transmitted and back scattered electromagnetic radiation of optical wave length range and recording maps of spatial distribution characterizing sequential and temporal continuous recordings of the transmitted and back scattered electromagnetic radiation.

36. An apparatus as described in claim 35, characterized by by the presence of a reference detector (13) of optical radiation optically connected with source (2) of the radiation and divisor (14), included between commutator (5) output and analog-to-digital converter (6) input, the input of reference detector (13) of optical radiation being output to the input of divisor (14).

37. An apparatus as described in claims 35, 36, characterized by containing sequentially connected sensor (19) of at least one of the parameters of the natural physiological living organism rhythms and temporal modulator (20), whose output is connected with optical radiation source (2) input and corresponding input of input-output controller (7).

38. An apparatus as described in claim 37, characterized by the realization of sensor (19) of natural physiological living organism rhythm via the mode of living organism respiration rhythm meter.

39. An apparatus as described in claim 37, characterized by the realization of sensor (19) of natural living organism physiological rhythm via the mode of cardiopulsation rhythm meter.

40. An apparatus as described in claim 37, characterized by realization of senior (19) of natural living organism physiological rhythm via the mode of muscle tremor meter.

41. An apparatus as described in any of claims 35-40, characterized by presence of optical system (24), located between source (2) of optical radiation and the surface of living organism (1), and focusing optical radiation at least into one point at living organism surface (1).

42. An apparatus as described in claim 41, characterized by containing a scanning system (29), optically connected with optical system (24), whose input is connected with the corresponding output of input-output controller (7).

43. An apparatus as described in any of claims 35 to 42, characterized by placing of at least one optical filter (34) at the same optical axis with source (2) of optical radiation between source (2) of optical radiation and the surface of living organism (1) illuminated.

44. An apparatus as described in any of claims 35-43, characterized by placing at least one additional optical filter (34) at the optical axis of detectors (3 and 4) of scattered by living organism radiation between the aforementioned detector and the surface of living organism investigated.

45. An apparatus as described in any of claims 35-44, characterized by the presence of frequency modulator (35), whose output is connected with source (2) of optical radiation and input - with corresponding output of input-output controller (7).

## Patentansprüche

1. Verfahren zur Diagnostik eines Zustandes eines lebenden Organismus mit folgenden Schritten:
- Bestrahlen des lebenden Organismus oder eines Teiles desselben mit elektromagnetischer Strahlung im optischen Wellenlängenbereich,
- Empfangen von durch den lebenden Organismus hindurchgegangener und rückgestreuter Strahlung des optischen Wellenlängenbereichs,
- Aufzeichnen derselben in Form von Abbildungen einer räumlichen Verteilung mindestens eines Parameters, der die Durchlässigkeit und die Rückstreuung elektromagnetischer Strahlung im optischen Wellenlängenbereich durch den lebenden Organismus kennzeichnet, wobei die räumliche Verteilung sequentiell und zeitlich kontinierlich aufgenommen wird.

2. Verfahren nach Anspruch 1, gekennzeichnet durch Aufnehmen der räumlichen Verteilung der Koeffizienten der Durchlässigkeit und der Rückstreuung der Strahlung durch den lebenden Organismus.

3. Verfahren nach Anspruch 1 oder 2, gekennzeichnet durch Amplitudenmodulation der Strahlung.

4. Verfahren nach einem der Ansprüche 1 bis 3, dadurch gekennzeichnet, daß die Amplitude der elektromagnetischen Strahlung synchron mit einem der physiologischen Rhythmen des lebenden Organismus stroposkopisch moduliert wird.

5. Verfahren nach einem der Ansprüche 1 bis 4, gekennzeichnet durch eine Konzentration elektromagnetischer Strahlung mindestens an einem Punkt einer Oberfläche des lebenden Organismus und Aufnehmen einer räumlichen Verteilung mindestens eines die Rückstreuung der elektromagnetischen Strahlung durch Gewebe des lebenden Organismus kennzeichnenden Parameters relativ dazu, wobei der Zustand von Geweben des Organismus in verschiedenen Tiefen beurteilt wird.

6. Verfahren nach Anspruch 5, gekennzeichnet durch Abtasten der bestrahlten Oberfläche des lebenden Organismus mit der auf mindestens einen Punkt konzentrierten elektromagnetischen Strahlung.

7. Verfahren nach einem der vorhergehenden Ansprüche, gekennzeichnet durch spektrales Filtern der durch den lebenden Organismus hindurchgegangenen und der vom lebenden Organismus zurückgestreuten elektromagnetischen Strahlung bei Absorptionsbanden physiologischer Pigmente.

8. Verfahren nach Anspruch 7, gekennzeichnet durch Frequenzfiltern der Strahlung mindestens in einem Bereich aus dem Wellenlängenintervall von 0,38 µm bis 0,48 µm und/oder von 0,52 µm bis 0,62 µm.

9. Verfahren nach Anspruch 7, gekennzeichnet durch Strahlungsfrequenzfilterung mindestens in einem Bereich aus dem Wellenlängenintervall von 0,6 µm bis 0,75 µm.

10. Verfahren nach Anspruch 7, gekennzeichnet durch Strahlungsfrequenzfilterung mindestens in einem Bereich aus dem Wellenlängenintervall von 1 µm bis 1,1 µm.

11. Verfahren nach einem der vorangegangenen Ansprüche, dadurch gekennzeichnet, daß die durch den lebenden Organismus hindurchgegangene und von dem lebenden Organismus rückgestreute, frequenzmodulierte elektromagnetische Strahlung synchron bei der Modulationsfrequenz empfangen wird.

12. Verfahren nach Anspruch 11, gekennzeichnet durch Modulation eines Strahls mindestens in einem Bereich aus dem Wellenlängenintervall von 0,6 µm bis 0,65 µm und/oder 0,65 µm bis 0,72 µm.

13. Verfahren nach einem der vorangegangenen Ansprüche, gekennzeichnet durch Sichtbar-Machen von räumlichen Bereichen in den sequentiell aufgenommenen räumlichen Verteilungen, wobei sich die räumlichen Bereiche voneinander mindestens in einem Parameter, der ihre zeitlichen Änderung kennzeichnet, unterscheiden, und durch Aufzeichnen der genannten Bereiche in Form einer funktionellen Abbildung in dem lebenden Organismus ablaufender physiologischer Prozesse.

14. Verfahren nach Anspruch 13, dadurch gekennzeichnet, daß als unterscheidender Parameter der zeitliche Kreuzkorrelationskoeffizient mindestens eines Parameters, der das Hindurchgehen und das Rückstreuen der elektromagnetischen Strahlung an unterschiedlichen Punkten des untersuchten Bereichs des lebenden Organismus kennzeichnet, verwendet wird.

15. Verfahren nach Anspruch 13, dadurch gekennzeichnet, daß bei Untersuchung wellenähnlicher physiologischer Prozesse als unterscheidender Parameter die Geschwindigkeit zeitlicher wellenähnlicher Änderungen mindestens eines Parameters, der das Hindurchgehen und die Rückstreuung der elektromagnetischen Strahlung durch den lebenden Organismus kennzeichnet, verwendet wird.

16. Verfahren nach Anspruch 13, dadurch gekennzeichnet, daß als unterscheidender Parameter eine Frequenz zeitlicher Änderungen mindestens eines Parameters, der das Hindurchgehen und die Rückstreuung elektromagnetischer Strahlung durch den lebenden Organismus kennzeichnet, verwendet wird.

17. Verfahren nach Anspruch 13, dadurch gekennzeichnet, daß als unterscheidender Parameter eine zeitliche Verzögerung von Änderungen mindestens eines Parameters, der das Hindurchgehen und die Rückstreuung genannten Strahlung durch den lebenden Organismus kennzeichnet, verwendet wird.

18. Verfahren nach Anspruch 13, dadurch gekennzeichnet, daß als unterscheidender Parameter Anzahl und Lage von Extrema in der zeitlichen Dynamik mindestens eines Parameters, der das Hindurchgehen und die Rückstreuung der genannten Strahlung durch den lebenden Organismus kennzeichnet, verwendet werden.

19. Verfahren nach einem der vorangegangenen Ansprüche, gekennzeichnet durch synchrones Aufnehmen mit mindestens einem der natürlichen physiologischen Rhythmen des lebenden Organismus.

20. Verfahren nach Anspruch 19, dadurch gekennzeichnet, daß der Atemrhythmus des lebenden Organismus als ein natürlicher physiologischer Rhythmus des lebenden Organismus verwendet wird.

21. Verfahren nach Anspruch 19, dadurch gekennzeichnet, daß der Herzschlagrhythmus des lebenden Organismus als ein natürlicher physiologischer Rhythmus des lebenden Organismus verwendet wird.

22. Verfahren nach Anspruch 19, dadurch gekennzeichnet, daß der Muskeltremor des lebenden Organismus als ein natürlicher physiologischer Rhythmus des lebenden Organismus verwendet wird.

23. Verfahren nach einem der vorhergehenden Ansprüche, gekennzeichnet durch simultane Untersuchung funktionell verbundener Bereiche des lebenden Organismus.

24. Verfahren nach einem der vorhergehenden Ansprüche, gekennzeichnet durch simultane Untersuchung paarweiser Organe des lebenden Organismus.

25. Verfahren nach einem der vorhergehenden Ansprüche, gekennzeichnet durch Anwenden mindestens einer äußeren Einwirkung auf den lebenden Organismus oder auf einen Teil des lebenden Organismus.

26. Verfahren nach Anspruch 25, dadurch gekennzeichnet, daß als äußere Einwirkung Änderungen physikalischer Parameter der den lebenden Organismus umgebenden Umgebung verwendet werden.

27. Verfahren nach Anspruch 25, dadurch gekennzeichnet, daß als äußere Einwirkung Änderungen in Parametern der externen Atmung des lebenden Organismus verwendet werden, und daß mittels erzielter optischer funktioneller Abbildungen, die physiologische Reaktionen des lebenden Organismus kennzeichnen, sowohl der Zustand des Atemsystems als auch der Zustand der Homöostase des gesamten Organismus beurteilt werden.

28. Verfahren nach Anspruch 25, gekennzeichnet durch die Verwendung von Vibration als ein äußerer Parameter.

29. Verfahren nach Anspruch 25, gekennzeichnet durch die Verwendung eines elektrischen Feldes als eine äußere Einwirkung.

30. Verfahren nach Anspruch 25, dadurch gekennzeichnet, daß ein auf verschiedene Bereich eines untersucht werdenden Organs einwirkendes magnetisches Wechselfeld als äußere Einwirkung verwendet wird und daß über die optischen funktionellen Abbildungen physiologischer Reaktionen auf solch eine Einwirkung an den reflektiven Hautprojektionen dieses Organs Vorhandensein und Lokalisation eines pathologischen Befundes beurteilt werden.

31. Verfahren nach Anspruch 26, gekennzeichnet durch die Verwendung von körperlicher Bewegung als äußere Einwirkung.

32. Verfahren nach Anspruch 26, dadurch gekennzeichnet, daß auf mindestens einen Punkt eines diagnostiziert werdenden inneren Organs fokussierter Ultraschall als äußere Einwirkung verwendet wird und daß Vorhandensein und Lokalisation pathologischer Befunde mittels der optischen funktionellen Abbildungen physiologischer Reaktionen auf diese Einwirkung an dem diesem Organ entsprechenden Dermatom beurteilt werden.

33. Verfahren nach Anspruch 25, dadurch gekennzeichnet, daß äußerer Druck als äußere Einwirkung verwendet wird und der Zustand diagnostiziert werdender Organe mittels optischer funktioneller Abbildungen physiologischer Reaktionen auf ein Anlegen und/oder Entfernen des äußeren Druckes beurteilt wird.

34. Verfahren nach Anspruch 25, gekennzeichnet durch die Verwendung von Wärmestrahlung und/oder Wärmeflüssen als äußere Einwirkung.

35. Vorrichtung zur Diagnostik eines Zustandes eines lebenden Organismus mit:
- einer Quelle (2) optischer Strahlung,
- einem auf der dem bestrahlten Teil des lebenden Organismus gegenüberliegenden Seite angeordneten Detektor (3) durch den lebenden Organismus (1) gestreuter Strahlung,
- einem Analog-Digital-Wandler (6), einem Eingangs-Ausgangs-Controller (7) und einem Computersystem (8), wobei der Analog-Digital-Wandler (6), der Eingangs-Ausgangs-Controller (7) und das Computersystem (8) in Reihe geschaltet sind,
- einem zusätzlichen, auf derselben Seite wie die optische Strahlungsquelle (2) angeordneten Detektor (4) durch den lebenden Organismus gestreuter Strahlung und
- einem Kommutator (5),
wobei zwei Eingänge des Kommutators (5) mit Ausgängen der entsprechenden Detektoren der durch den lebenden Organismus gestreuten Strahlung verbunden sind, der Ausgang des Kommutators (5) mit einem Eingang des Analog-Digital-Wandlers (6) verbunden ist, drei Ausgänge des Eingangs-Ausgangs-Controllers entsprechend mit einem Steuereingang des Kommutators und des jeweiligen Detektors der durch den lebenden Organismus gestreuten Strahlung verbunden sind und die Vorrichtung hindurchgehende und rückgestreute elektromagnetische Strahlung im optischen Wellenlängenbereich aufnimmt und Abbildungen räumlicher Verteilung, die sequentiell und zeitlich kontinuierliche Aufnahmen der hindurchgegangenen und der rückgestreuten elektromagnetischen Strahlung beschreibt, aufzeichnet.

36. Vorrichtung nach Anspruch 35, gekennzeichnet durch einen optisch mit der Strahlungsquelle (2) verbundenen Referenzdetektor (13) optischer Strahlung und einen zwischen einem Ausgang des Kommutators (5) und einem Eingang des Analog-Digital-Wandlers (6) angeordneten Teiler (14), wobei ein Eingang des Referenzdetektors (13) optischer Strahlung mit einem Eingang des Teilers (14) verbunden ist.

37. Vorrichtung nach Anspruch 35 oder 36, gekennzeichnet durch eine Reihenschaltung aus einem Sensor (19) mindestens eines Parameters des natürlichen physiologischen Rhythmus des lebenden Organismus und einem Zeitmodulator (20), dessen Ausgang mit einem Eingang der optischen Strahlungsquelle (2) und einem entsprechenden Eingang des Eingangs-Ausgangs-Controllers (7) verbunden ist.

38. Vorrichtung nach Anspruch 37, dadurch gekennzeichnet, daß der Sensor (19) eines natürlichen physiologischen Rhythmus des lebenden Organismus als Atemrhythmusmesser ausgebildet ist.

39. Vorrichtung nach Anspruch 37, dadurch gekennzeichnet, daß der Sensor (19) eines natürlichen physiologischen Rhythmus des lebenden Organismus als Herzschlagrhythmusmesser ausgebildet ist.

40. Vorrichtung nach Anspruch 37, dadurch gekennzeichnet, daß der Sensor (19) eines natürlichen physiologischen Rhythmus des lebenden Organismus als Muskeltremormesser ausgebildet ist.

41. Vorrichtung nach einem der Ansprüche 35 bis 40, gekennzeichnet durch ein optisches System (24), das zwischen der Quelle (2) optischer Strahlung und der Oberfläche des lebenden Organismus (1) angeordnet ist, und optische Strahlung mindestens in einem Punkt auf der Oberfläche des lebenden Organismus (1) fokussiert.

42. Vorrichtung nach Anspruch 41, gekennzeichnet durch ein Abtastsystem (29), das optisch mit dem optischen System (24) verbunden ist und dessen Eingang mit dem entsprechenden Ausgang des Eingangs-Ausgangs-Controllers (7) verbunden ist.

43. Vorrichtung nach einem der Ansprüche 35 bis 42, dadurch gekennzeichnet, daß mindestens ein optisches Filter (34) auf derselben optischen Achse wie die Quelle (2) optischer Strahlung zwischen der Quelle (2) optischer Strahlung und der bestrahlten Oberfläche des lebenden Organismus (1) angeordnet ist.

44. Vorrichtung nach einem der Ansprüche 35 bis 43, dadurch gekennzeichnet, daß mindestens ein zusätzliches optisches Filter (34) auf der optischen Achse von Detektoren (3 und 4) durch den lebenden Organismus gestreuter Strahlung zwischen dem eben genannten Detektor und der untersucht werdenden Oberfläche des lebenden Organismus angeordnet ist.

45. Vorrichtung nach einem der Ansprüche 35 bis 44, gekennzeichnet durch einen Frequenzmodulator (35), dessen Ausgang mit der Quelle (2) optischer Strahlung und dessen Eingang mit einem entsprechenden Ausgang des Eingangs-Ausgangs-Controllers (7) verbunden ist.

## Revendications

1. Procédé pour le diagnostic de l'état d'un organisme vivant comprenant les étapes suivantes :
illuminer l'organisme vivant ou une partie de celui-ci avec un rayonnement électromagnétique dans la gamme de longueurs d'onde optique,
capter le rayonnement émis à travers l'organisme vivant et rétrodiffusé de la gamme de longueurs d'onde optique,
l'enregistrer sous la forme de cartes de répartition spatiale d'au moins un paramètre, caractérisant la transmittance et la rétrodiffusion par l'organisme vivant du rayonnement électromagnétique de la gamme de longueurs d'onde optique, la répartition spatiale susmentionnée étant continuellement enregistrée de manière séquentielle et temporelle.

2. Procédé selon la revendication 1, caractérisé par l'enregistrement de la répartition spatiale des coefficients de transmittance et de rétrodiffusion du rayonnement susmentionné par l'organisme vivant.

3. Procédé selon les revendications 1, 2, caractérisé par la modulation d'amplitude du rayonnement susmentionné.

4. Procédé selon les revendications 1 à 3, caractérisé en ce que le rayonnement électromagnétique susmentionné est modulé stroboscopiquement en amplitude en synchronisation avec un des rythmes physiologiques de l'organisme vivant.

5. Procédé selon les revendications 1 à 4, caractérisé par une concentration du rayonnement électromagnétique sur au moins un point de la surface de l'organisme vivant et l'enregistrement d'une répartition radiale y relatif d'au moins un paramètre, caractérisant la rétrodiffusion de ce rayonnement électromagnétique par les tissus de l'organisme vivant et permettant ainsi d'évaluer l'état des tissus de l'organisme à différentes profondeurs.

6. Procédé selon la revendication 5, caractérisé par le balayage de la surface éclairée de l'organisme vivant avec le rayonnement électromagnétique susmentionné, concentré sur au moins un point.

7. Procédé selon l'une quelconque des revendications précédentes, caractérisé par le filtrage spectral de l'illumination du rayonnement électromagnétique d'un organisme vivant émis et rétrodiffusé par ce dernier au niveau des bandes d'absorption des pigments physiologiques.

8. Procédé selon la revendication 7, caractérisé par un filtrage de fréquence du rayonnement au niveau d'au moins une bande de l'intervalle de longueur d'onde de 0,38 µm à 0,48 µm et/ou de 0,52 µm à 0,62 µm.

9. Procédé selon la revendication 7, caractérisé par le filtrage de la fréquence de rayonnement au niveau d'au moins une bande de l'intervalle de longueur d'onde de 0,6 µm à 0,75 µm.

10. Procédé selon la revendication 7, caractérisé par le filtrage de la fréquence de rayonnement au niveau d'au moins une bande de l'intervalle de longueur d'onde de 1 µm à 1,1 µm.

11. Procédé selon l'une quelconque des revendications précédentes, caractérisé en ce que la fréquence modulée émise et rétrodiffusée par le rayonnement électromagnétique de l'organisme vivant est reçue en synchronisation avec la fréquence de modulation.

12. Procédé selon la revendication 11, caractérisé par la modulation d'un faisceau au niveau d'au moins une bande de l'intervalle de longueur d'onde de 0,6 µm à 0,65 µm et/ou de 0,65 à 0,72 µm.

13. Procédé selon l'une quelconque des revendications précédentes, caractérisé par la recherche, dans des répartitions spatiales susmentionnées enregistrées séquentiellement, de régions spatiales qui diffèrent entre elles par au moins un paramètre, caractérisant leur modification temporelle, et par l'enregistrement des régions ci-dessus sous la forme d'une carte fonctionnelle de processus physiologiques se produisant dans l'organisme vivant.

14. Procédé selon la revendication 13, caractérisé par l'utilisation, comme paramètre distinctif, du coefficient d'intercorrélation temporelle d'au moins un paramètre, caractérisant la transmittance et la rétrodiffusion du rayonnement électromagnétique susmentionné à différents points de la région de l'organisme vivant étudié.

15. Procédé selon la revendication 13, caractérisé par l'utilisation, comme paramètre distinctif, à l'étude de processus physiologiques semblables à une onde, de la vitesse des changements temporaires semblables à une onde d'au moins un paramètre, caractérisant la transmittance et la rétrodiffusion du rayonnement électromagnétique par l'organisme vivant.

16. Procédé selon la revendication 13, caractérisé par l'utilisation, comme paramètre distinctif, d'une fréquence de changements temporaires d'au moins un paramètre, caractérisant la transmittance et la rétrodiffusion du rayonnement électromagnétique par l'organisme vivant

17. Procédé selon la revendication 13, caractérisé par l'utilisation, comme paramètre distinctif, d'un temps de retard de changements d'au moins un paramètre, caractérisant la transmittance et la rétrodiffusion du rayonnement susmentionné par l'organisme vivant.

18. Procédé selon la revendication 13, caractérisé par l'utilisation, comme paramètre distinctif, du nombre et de la position des extrêmes à la dynamique temporelle d'au moins un paramètre, caractérisant la transmittance et la rétrodiffusion du rayonnement susmentionné par l'organisme vivant.

19. Procédé selon l'une quelconque des revendications précédentes, caractérisé par un enregistrement synchronisé avec au moins un des rythmes physiologiques de l'organisme vivant naturel.

20. Procédé selon la revendication 19, caractérisé par l'utilisation du rythme respiratoire de l'organisme vivant, comme rythme physiologique naturel de l'organisme vivant.

21. Procédé selon la revendication 19, caractérisé par l'utilisation du rythme cardiaque de l'organisme vivant, comme rythme physiologique naturel de l'organisme vivant.

22. Procédé selon la revendication 19, caractérisé par l'utilisation des contractions musculaires de l'organisme vivant, comme rythme physiologique naturel de l'organisme vivant.

23. Procédé selon l'une quelconque des revendications précédentes, caractérisé par une étude simultanée des régions fonctionnellement connectées de l'organisme vivant.

24. Procédé selon l'une quelconque des revendications précédentes, caractérisé par l'étude simultanée d'organes appariés de l'organisme vivant.

25. Procédé selon l'une quelconque des revendications précédentes, caractérisé par l'application d'au moins une influence extérieure sur l'organisme vivant ou une partie de celui-ci.

26. Procédé selon la revendication 25, caractérisé par l'utilisation, comme influence extérieure, de changements dans les paramètres physiques de l'environnement, entourant l'organisme vivant.

27. Procédé selon la revendication 25, caractérisé par l'utilisation, comme influence extérieure, de changements dans les paramètres de la respiration externe de l'organisme vivant, et permettant par l'intermédiaire de cartes fonctionnelles optiques obtenues, caractérisant des réactions physiologiques de l'organisme vivant, d'évaluer l'état à la fois du système respiratoire et de l'homéostasie de l'organisme complet.

28. Procédé selon la revendication 25, caractérisé par l'utilisation de vibrations comme paramètre externe.

29. Procédé selon la revendication 25, caractérisé par l'utilisation d'un champ électrique, comme influence extérieure.

30. Procédé selon la revendication 25, caractérisé par l'utilisation, comme influence extérieure, d'un champ magnétique alternatif affectant différentes régions d'un organe étudié et permettant, par l'intermédiaire des cartes fonctionnelles optiques de réactions physiologiques sur une telle influence aux projections épidermiques réfléchissantes de cet organe, d'évaluer la présence et la position de la pathologie.

31. Procédé selon la revendication 26, caractérisé par l'utilisation d'exercices physiques, comme influence extérieure.

32. Procédé selon la revendication 26, caractérisé par l'utilisation, comme influence extérieure, d'un signal ultrasonore concentré sur au moins un point de l'organe interne diagnostiqué, la présence et la position de pathologies étant évaluées par les cartes fonctionnelles optiques de réactions physiologiques sur cette influence au dermatome correspondant à cet organe.

33. Procédé selon la revendication 25, caractérisé par l'utilisation d'une pression externe comme influence extérieure, l'état des organes diagnostiqués étant évalué à l'aide des cartes fonctionnelles optiques de réactions physiologiques à son application et/ou sa suppression.

34. Procédé selon la revendication 25, caractérisé par l'utilisation d'un rayonnement thermique et/ou d'écoulements thermiques, comme influence extérieure.

35. Dispositif pour le diagnostic de l'état d'un organisme vivant contenant : une source (2) de rayonnement optique, un détecteur (3) de rayonnement diffusé par l'organisme vivant (1) situé du côté opposé à la partie illuminée de l'organisme vivant (1), un convertisseur analogique-numérique connecté en séquence (6), un contrôleur d'entrée/sortie (7), un ordinateur (8), un détecteur supplémentaire (4) du rayonnement diffusé par l'organisme vivant du même côté que la source de rayonnement optique (2), un commutateur (5), dont deux entrées sont connectées aux sorties des détecteurs correspondants du rayonnement diffusé par l'organisme vivant, tandis que la sortie est connectée à l'entrée du convertisseur analogique-numérique (6), trois sorties du contrôleur d'entrée/sortie étant connectées aux entrées de commande correspondantes du commutateur et du détecteur de rayonnement diffusé par l'organisme vivant, ledit appareil enregistrant le rayonnement électromagnétique rétrodiffusé de la gamme de longueurs d'onde optique et enregistrant des cartes de la répartition spatiale caractérisant les enregistrements continus séquentiels et temporels du rayonnement électromagnétique émis et rétrodiffusé.

36. Dispositif selon la revendication 35, caractérisé par la présence d'un détecteur de référence (13) de rayonnement optique connecté optiquement avec la source de rayonnement (2) et le diviseur (14), placé entre la sortie du commutateur (5) et l'entrée du convertisseur analogique-numérique (6), l'entrée du détecteur de référence (13) du rayonnement optique étant connectée à l'entrée du diviseur (14).

37. Dispositif selon les revendications 35, 36, caractérisé en ce qu'il contient un détecteur connecté en séquence (19) d'au moins un des paramètres des rythmes physiologiques naturels de l'organisme vivant et d'un modulateur temporel (20) dont la sortie est connectée à l'entrée de la source de rayonnement optique (2) et à l'entrée correspondante du contrôleur d'entrée/sortie (7).

38. Dispositif selon la revendication 37, caractérisé par la réalisation du détecteur (19) du rythme physiologique naturel de l'organisme vivant par l'intermédiaire du mode de mesure du rythme respiratoire de l'organisme vivant.

39. Dispositif selon la revendication 37, caractérisé par la réalisation du détecteur (19) du rythme physiologique naturel de l'organisme vivant par l'intermédiaire du mode de mesure du rythme cardiaque.

40. Dispositif selon la revendication 37, caractérisé par la réalisation du détecteur (19) du rythme physiologique naturel de l'organisme vivant par l'intermédiaire du mode de mesure des contractions musculaires.

41. Dispositif selon l'une quelconque des revendications 35 à 40, caractérisé par la présence du système optique (24) situé entre la source de rayonnement optique (2) et la surface de l'organisme vivant (1), et concentrant le rayonnement optique sur au moins un point à la surface de l'organisme vivant (1).

42. Dispositif selon la revendication 41, caractérisé par un système d'analyse (29) connecté optiquement au système optique (24), dont l'entrée est connectée à la sortie correspondante du contrôleur d'entrée/sortie (7).

43. Dispositif selon l'une quelconque des revendications 35 à 42, caractérisé par l'installation d'au moins un filtre optique (34) dans le même axe optique que la source de rayonnement optique (2) entre la source de rayonnement optique (2) et la surface illuminée de l'organisme vivant (1).

44. Dispositif selon l'une quelconque des revendications 35 à 43, caractérisé par l'installation d'au moins un filtre optique supplémentaire (34) dans l'axe optique des détecteurs (3 et 4) de rayonnement diffusé par l'organisme vivant entre le détecteur susmentionné et la surface de l'organisme vivant analysé.

45. Dispositif selon l'une quelconque des revendications 35 à 44, caractérisé par la présence d'un modulateur de fréquence (35) dont la sortie est connectée à la source de rayonnement optique (2) et l'entrée est connectée à la sortie correspondante du contrôleur d'entrée/sortie (7).
